**Europäisches Patentamt**

**European Patent Office**

**Office européen des brevets**

(19)

(11) Veröffentlichungsnummer: **0 200 106**
**B1**

(12) # EUROPÄISCHE PATENTSCHRIFT

(45) Veröffentlichungstag der Patentschrift:
**27.12.90**

(21) Anmeldenummer: **86105328.8**

(22) Anmeldetag: **17.04.86**

(51) Int. Cl.⁵: **C07C 51/02, C07C 59/265,**
**C07C 229/00, C07C 303/00**

(54) Verfahren zur Gewinnung von wässrigen Lösungen organischer Säuren oder Basen aus wässrigen Lösungen ihrer Salze.

(30) Priorität: **20.04.85 DE 3514348**

(43) Veröffentlichungstag der Anmeldung:
**10.12.86 Patentblatt 86/45**

(45) Bekanntmachung des Hinweises auf die Patenterteilung:
**27.12.90 Patentblatt 90/52**

(84) Benannte Vertragsstaaten:
**DE FR GB IT**

(56) Entgegenhaltungen:
**DE-A- 1 014 980**
**FR-A- 2 126 586**
**US-A- 4 212 994**

**INDUSTRIAL AND ENGINEERING CHEMISTRY, Band 50,**
**Nr. 8, August 1958, Seiten 1181-1188; G.W. MURPHY:**
**"Osmionic demineralization"**

(73) Patentinhaber: **BASF Aktiengesellschaft,**
**Cari-Bosch-Strasse 38, D-6700 Ludwigshafen(DE)**

(72) Erfinder: **Puetter, Hermann, Dr., Haardter Strasse 1,**
**D-6730 Neustadt(DE)**
Erfinder: **Voss, Hartwig, Dr., Mozartstrasse 57,**
**D-6710 Frankenthal(DE)**

## Beschreibung

Diese Erfindung betrifft ein Verfahren zur Gewinnung von wäßrigen Lösungen organischer Säuren oder Basen aus wäßrigen Lösungen ihrer Salze durch Ionenaustausch in einer Austauschzelle.

Häufig fallen Säuren oder Basen bei der Synthese in Form von wäßrigen Lösungen ihrer Salze an. So verläuft z.B. die Streckersynthese zur Herstellung von Aminosäuren in der Endstufe über eine alkalische oder saure Hydrolyse eines Aminonitrils, so daß die Aminosäure entweder als Salz eines Alkali- oder Erdalkalimetalls oder als Salz einer starken Mineralsäure anfällt. Um die Aminosäure in freier (Betain-)Form zu gewinnen, müssen also diese Salze mit der Gruppierung -COO$^\ominus$M$^\oplus$ bzw. -NH$_3$$^\oplus$ X$^\ominus$ (M$^\oplus$ = Metallkation, X$^\ominus$ = Säureanion) zunächst mit einer anorganischen Base oder Mineralsäure behandelt werden.

Auch bei der Synthese anderer organischer Säuren, die gut wasserlöslich sind, wie Hydroxycarbonsäuren, welche man z.b. durch mikrobiologische Verfahren herstellt, sowie auch bei der Rückgewinnung von organischen Hilfsbasen, wie Trimethylamin, fallen wäßrige Salzlösungen an, aus denen die freien Basen oder Säuren nur schwierig zu isolieren sind. Als weitere technische Schwierigkeit kommt hinzu, daß man die freien Säuren oder Basen von den ebenfalls gut löslichen Neutralsalzen abtrennen muß. Man kann dies erreichen durch den Einsatz von Ionenaustauschern oder mit Hilfe der Elektrolyse oder Elektrodialyse.

Diese Methoden haben jedoch Nachteile. So benötigt man bei den Ionenaustauscherverfahren stöchiometrische Mengen des entsprechenden Harzes in H$^\oplus$-Form bzw. OH$^\ominus$-Form. Dieser große Mengenbedarf an Ionenaustauschermasse ist unwirtschaftlich, er führt auch zu Produktverlusten oder zu starker Verdünnung des Wertproduktes. Bei der Elektrolyse, die man z.B. in sogenannten Dreikammerzellen durchführt, muß man pro Neutralisationsäquivalent mindestens 1 Faraday an Stromeinsatz aufwenden. Der elektrische Energieverbrauch ist erheblich. Bei der Elektrodialyse mit bipolaren Membranen spart man gegenüber der Elektrolyse zwar Elektrodenfläche und einen Teil der notwendigen Energie ein, der elektrische Energieverbrauch ist aber dennoch hoch, da zusätzlich zur Überwindung des elektrischen Widerstandes Energie für die Wasserdissoziation benötigt wird.

Es wurde nun gefunden, daß man wäßrige Lösungen organischer Säuren oder Basen aus den wäßrigen Lösungen ihrer Salze erheblich vorteilhafter gewinnen kann, wenn man

a) eine wäßrige Lösung eines Salzes einer organischen Säure oder einer organischen Base und
b) eine wäßrige Lösung einer anorganischen Base oder einer Säure

jeweils in, durch eine Ionenaustauschermembran voneinander getrennte, Kammern einer Austauschzelle einleitet und die Lösungen (a) und (b) an der Oberfläche der Membran vorbeiführt.

Mit dem erfindungsgemäßen Verfahren werden die geschilderten Nachteile vermieden. Anstelle stöchiometrischer Mengen an Ionenaustauschermasse werden Ionenaustauschermembranen benutzt, so daß sowohl ein Produktverlust als auch eine Verdünnung der Wertprodukte vermieden wird. Außerdem kommt das erfindungsgemäße Verfahren ohne Elektrolyse- bzw. Elektrodialysestrom aus.

Das erfindungsgemäße Verfahren führt zur Freisetzung von organischen Säuren oder Basen mit Hilfe von Ionenaustauschermembranen, durch die hindurch die störenden anorganischen Ionen M$^\oplus$ bzw. X$^\ominus$ gegen H$^\oplus$ bzw. OH$^\ominus$ ausgetauscht werden. Im Gegensatz zum Ionentransport mit Flüssigmembranen, bei dem ebenfalls ein M$^\oplus$/H$^\oplus$-Austausch erfolgt, wird hier keine Carrierverbindung, die erst den Transport ermöglicht, benötigt.

Es ist zwar z.B. aus der EP-PS 126 830 bekannt, daß man schwer lösliche Salze anionischer Farbstoffe durch Ionenaustausch über eine Ionenaustauschermembran in leichter lösliche Salze überführen kann. Es war aber nicht zu erwarten, daß man nach dem Verfahren der vorliegenden Erfindung die freien Säuren bzw. Basen aus ihren Salzen auf so vorteilhafte Weise gewinnen kann, zumal das erfindungsgemäße Verfahren das bekannte Ionenaustauschverfahren in der Geschwindigkeit des Austausches erheblich übertrifft.

Als Salze organischer Säuren, die man nach dem erfindungsgemäßen Verfahren in die freien Säuren überführt, kommen z.B. wasserlösliche Alkali-, Erdalkali-, Kupfer-, Eisen-, Zink- oder Chromsalze von organischen Säuren in Betracht. Geeignete organische Säuren sind z.B. Carbonsäuren, Hydroxycarbonsäuren, Aminosäuren, Phosphonsäuren oder in wäßrigem Medium sauer reagierende organische Verbindungen, wie Phenole, Amide, Sulfonamide oder Sulfonylamide. Bevorzugt sind Salze von Säuren mit einer Löslichkeit in Wasser von mindestens 5, vorzugsweise mindestens 10 Gew.%. Die organischen Säuren können ein- oder mehrwertig sein, ihr pK$_S$-Wert liegt vorzugsweise bei >2. So kommen z.B. Dicarbonsäuren oder Polycarbonsäuren in Betracht. Von besonderem technischen Interesse ist die erfindungsgemäße Gewinnung von wasserlöslichen Hydroxycarbonsäuren, wie Milchsäure, Citronensäure oder Zuckersäuren oder von Aminosäuren, wie Glycin, α- und β-Alanin, N-alkylierten Derivaten des Glyc ins oder Alanins, Aminobuttersäuren, Karnitin oder Anthranilsäuren. Säuren mit Molekulargewichten von <200 sind bevorzugt.

Als Salze organischer Basen kommen z.B. wasserlösliche Salze aus Mineralsäuren, wie Salzsäure, Schwefelsäure oder Phosphorsäure einerseits und ein- oder mehrwertigen organischen Basen andererseits, wie Aminen, basisch wirkenden Aminosäuren, heterocyclischen Verbindungen oder quaternäre Ammoniumverbindungen in Betracht. Von besonderem technischen Interesse ist die erfindungsgemäße Gewinnung von wasserlöslichen organischen Basen, wie Trialkylamin (Alkyl mit 1 bis 4 C-Atomen) oder von Hilfsbasen, wie Diazabicyclooctan oder Dicyclohexylethylamin oder von polymeren Aminen, wie Polyethyliminen oder Polyvinylaminen. Als weitere bevorzugte Basen seien z.B. Aminosäuren, wie die oben erwähnten oder quaternäre Ammoniumverbindungen genannt.

Salze von mehrbasischen Säuren können nach dem neuen Verfahren auch stufenweise in die freien Säuren übergeführt werden. So kann man z.B. eine tribasische Salzverbindung, wie das Trinatriumcitrat mit hoher Selektivität in die Dinatriumhydrogenverbindung bzw. in die Mononatriumdihydrogen-Verbindung überführen. Dies gilt in gleicher Weise auch für die Salze mehrwertiger Basen, die man entsprechend selektiv stufenweise in die jeweiligen freien Basen überführen kann. Die Salze der organischen Säuren oder Basen werden in den wäßrigen Lösungen z.B. in Konzentrationen von 1 bis 40 Gew.% eingesetzt. Die Basen haben einen $pK_B$-Wert, der vorzugsweise bei >2 liegt.

Da die Freisetzung der organischen Säuren aus ihren Salzen auf dem Austausch gleichsinnig geladener Ionen zwischen zwei wäßrigen Lösungen durch die Ionenaustauschermembran beruht, benötigt man neben der Prozeßlösung (a), die das Salz der organischen Säure enthält, eine Hilfslösung (b), die eine Säure, wie eine anorganische Säure oder eine Sulfonsäure enthält. Im Fall der Basenfreisetzung enthält die Prozeßlösung (a) das Salz der Base und die Hilfslösung (b) eine anorganische Base.

Anorganische Säuren sind z.B. Mineralsäuren, wie Salz- oder Schwefelsäure. Anorganische Basen sind z.B. anorganische Verbindungen, die $OH^\ominus$-Gruppen enthalten oder in Wasser freisetzen, wie die Hydroxide oder Carbonate der Alkali- oder Erdalkalimetalle oder des Ammoniums. Bevorzugt sind hier NaOH, KOH, $Na_2CO_3$, $K_2CO_3$, $Ca(OH)_2$, $Ba(OH)_2$, $NH_4OH$ oder $(NH_4)_2CO_3$. Die genannten Säuren bzw. Basen werden in der wäßrigen Hilfslösung z.B. in einer Konzentration von 0,1 bis 10 Äquivalenten pro Liter angewendet. Man verwendet dabei zweckmäßigerweise mindestens eine stöchiometrische Menge der Säure bzw. der Base, bezogen auf die freizusetzende organische Säure oder Base. Zweckmäßigerweise verwendet man die 1 bis 10fache, vorzugsweise die 1 bis 3fache stöchiometrische Menge.

Das Verfahren wird in einer Austauschzelle durchgeführt, die mindestens zwei durch eine Ionenaustauschermembran getrennte Räume enthält, so daß zwei voneinander getrennte Flüssigkeitsströme möglich sind. Austauschzellen dieser Art werden z.B. für die bekannten Verfahren der Elektrodialyse verwendet, wobei im vorliegenden Fall jedoch die Elektrodenkammern entfallen, da kein elektrisches Feld benötigt wird. Eine geeignete Apparatur wird z.B. in der EP-PS 126 830 beschrieben. Gut geeignet sind als Austauschzellen z.B. mit Membranstapeln ausgerüstete Apparate, die eine Vielzahl, wie 2 bis 800 parallel zueinander angeordnete Kammern enthalten. Da kein elektrisches Feld anzulegen ist, ist man jedoch nicht an diese sogenannten Platten-Membranmodule gebunden. Alle anderen Austauschzellen sind ebenfalls einsetzbar, wie Hohlfaser-, Rohr- oder Wickelmodule. Die Kammern der Austauschzellen, die alternativ mit den wäßrigen Lösungen (a) und (b) beschickt werden, sind zum Zwecke der Beströmung der Membranen, z.B. mit externen Vorratsgefäßen, verbunden. Man kann das Verfahren kontinuierlich oder diskontinuierlich durchführen. Beim Batchverfahren erfolgt ein mehrfacher und beim kontinuierlichen Betrieb ein einmaliger Durchlauf der Lösungen durch die Austauschzelle. Die beiden Lösungen können dabei im Parallel-, Kreuz- oder Gegenstrom durch die Austauschzelle geleitet werden. Weitere Austauschzellen können in Form einer mehrstufigen Kaskade angeordnet sein, insbesondere beim kontinuierlichen Betrieb.

Als Ionenaustauschermembranen kommen an sich bekannte permselektive Kationenaustauschermembranen oder Anionenaustauschermembranen in Betracht, die z.B. eine Dicke von 0.1 bis 1 mm und einen Porendurchmesser von 1 bis 30 µm bzw. eine gelartige Struktur aufweisen. Membranen dieser Art sind z.B. unter den Bezeichnungen ®Selemion, ®Neosepta oder ®Nafion im Handel erhältlich. Da es sich um einen Diffusionsprozeß handelt, werden besonders dünne Membranen, z.B. solche mit einer Dicke von <0,2 mm bevorzugt. Zur Freisetzung der organischen Säuren verwendet man Kationenaustauschermembranen, während der Ionenaustausch bei der Freisetzung der Basen Anionenaustauschermembranen erfordert.

Das Verfahren wird bei Temperaturen von -20 bis +80°C, vorzugsweise +10 bis +50°C, und bei Drücken von 1 bis 10 bar, vorzugsweise bei Atmosphärendruck durchgeführt. Der Druckabfall über die eingesetzten Membranen beträgt 0 bis 5 bar, insbesondere 0 bis 0,2 bar.

Das erfindungsgemäße Verfahren ermöglicht es, auf wirtschaftlich vorteilhafte Weise wasserlösliche Säuren und Basen aus ihren Salzen freizusetzen, wobei man wäßrige Lösungen der Säuren oder Basen erhält, die frei von unerwünschten anorganischen Stoffen sind.

Das Verfahren der Erfindung zeichnet sich außerdem durch unerwartet hohe Austauschraten aus.

Beispiel 1

Gewinnung von Citronensäure aus dem Trinatriumsalz

Als Austauschzelle wurde ein Zweikreis-Membranstapel verwendet, wie er üblicherweise für Elektrodialysen herangezogen wird, der in diesem Fall jedoch keine Elektrodenkammern aufwies. Als Mineralsäure wurde Salzsäure verwendet. Die Trinatriumcitrat-Lösung und die wäßrige Salzsäure wurden alternierend durch die parallel angeordnete Kammern des Membranstapels im Kreis über jeweils ein externes Vorratsgefäß gepumpt. Der Membranstapel enthielt nur Kationenaustauschermembranen, durch die der Austausch von Natriumionen gegen Wasserstoffionen vonstatten ging. Der Fortgang der im Batchbetrieb durchgeführten Umwandlung wurde über Leitfähigkeits- und pH-Wertmessung kontrolliert.

Einzelheiten der Verfahrensführung sind aus der nachfolgenden Zusammenstellung ersichtlich:

```
Prozeßführung: Batchbetrieb (Austausch Na⊕ gegen H⊕)
Membranen:     Im Handel unter der Bezeichnung ®Selemion CMV erhältliche
               Kationenaustauschermembran mit der Oberfläche 0,15 m²
Temperatur:    25°C
Prozeßkreis:   Eintrag:   20 1 einer 0,05 molaren wäßrigen Trinatrium-
                          citratlösung;
               Austrag:   20 1 einer wäßrigen Lösung mit einer Wasser-
                          stoffionenkonzentration von 0,148 mol/1 und
                          einer Citronensäurekonzentration von
                          0,0507 mol/1 (Umsetzungsgrad 97 %)
Hilfskreis:    Eintrag: . 35 1 0,2 molare Salzsäure
                          (1,3facher stöchiometrischer Überschuß)
Austauschrate
[mol/m²·Tag]:  Mittel = 6, Maximum = 126
```

Beispiel 2

Beispiel 1 wurde wiederholt, wobei der Versuch jedoch zur Gewinnung der ein-, zwei- und dreibasischen Säure nach Erreichen des entsprechenden Umsatzgrades (33 bzw. 66 bzw. 100 %) abgebrochen wurde.

Die mittlere Austauschrate [mol/m²•Tag] betrug bei den drei Stufen:

| 1 | 2 | 3 |
|---|---|---|
| Dinatriumcitrat | Mononatriumcitrat | Citronensäure |
| 123 . | 117 | 47 |

Beispiel 3

Gewinnung von Bis-tetrabutylammoniumsulfat (BTBAS) aus Tetrabutylammoniumhydrogensulfat (TBAHS) unter Verwendung des in Beispiel 1 beschriebenen Zweikreis-Membranstapels.

Einzelheiten der Verfahrensführung:

```
Prozeßführung: Batchbetrieb (Austausch HSO₃⊖ gegen OH⊖)
Membranen:     Im Handel unter der Bezeichnung ®Selemion DMV erhältliche
               Anionenaustauschermembran mit der Oberfläche 0,037 m²
Temperatur:    20 bis 25°C
Prozeßkreis:   Eintrag:   Eine Lösung von 173,5 g (0,51 Mol) TBAHS in
                          2000 g Wasser
               Austrag:   2004 g (ohne Proben) einer wäßrigen Lösung, die
                          0,51 mol BTBAS enthält (Umsatzgrad 100 %)
Hilfskreis:    Eintrag:   2 kg einer 1 n Natronlauge (2,9facher
                          stöchiometrischer Überschuß)
Austauschrate
[mol/m²·Tag]:  im Mittel: 110
               Maximum:   250
```

Beispiel 4

Gewinnung des Mononatriumsalzes der Iminodiessigsäure (IDSHNa) aus dem Dinatriumsaz der Iminodiessigsäure (IDSNa₂) unter Verwendung des in Beispiel 1 beschriebenen Zweikreis-Membranplattenstapels.

. Einzelheiten der Verfahrensführung:

```
Prozeßführung: Batchbetrieb (Austausch Na⊕ gegen H⊕)
Membranen:     Kationenaustauschermembran wie in Beispiel 1 beschrieben
               mit einer Oberfläche von 0,02 m².
Temperatur:    20 bis 25°C
Prozeßkreis:   Eintrag:   1800 g einer wäßrigen Lösung von 2,41 mol
                          IDSNa₂, die 0,2 mol NaOH enthielt
               Austrag:   1650 g (ohne Proben) einer wäßrigen Lösung, die
                          2,35 mol IDSHNa enthält
Hilfskreis:    2 kg einer 20 %igen Schwefelsäure (2,4-facher stöchio-
               metrischer Überschuß)
Austauschrate
[mol/m²·Tag]:  im Mittel 80
```

Beispiel 5

Gewinnung von γ-Aminobuttersäure aus ihrem Hydrochlorid unter Verwendung des in Beispiel 1 beschriebenen Zweikreis-Membranstapels.

Einzelheiten der Verfahrensführung:

```
Prozeßführung: Batchbetrieb (Austausch Cl⊖ gegen OH⊖)
Membran:       Anionenaustauschermembran wie in Beispiel 3 beschrieben
               mit einer Oberfläche von 0,037 m².
Temperatur:    30°C
Prozeßkreis:   Eintrag:   Eine Lösung von 69,8 g (0,5 mol)
                          γ-Aminobuttersäure-Hydrochlorid in 2031 g
                          Wasser
               Austrag:   1860 g (ohne Proben) einer wäßrigen Lösung, die
                          0,5 mol γ-Aminobuttersäure enthält, mit einem
                          Cl⊖-Gehalt < 0,01 %.
Hilfskreis:    2 kg 1 n NaOH (3-facher stöchiometrischer Überschuß)
Austauschrate: Mittel = 108
[mol/m²·Tag]:  Maximum = 170
```

**Patentansprüche**

1. Verfahren zur Gewinnung von wäßrigen Lösungen organischer Säuren oder Basen, dadurch gekennzeichnet, daß man
a) eine wäßrige Lösung eines Salzes einer organischen Säure oder einer organischen Base und
b) eine wäßrige Lösung einer anorganischen Base oder einer Säure
jeweils in, durch eine Ionenaustauschermembran voneinander getrennte, Kammern einer Austauschzelle einleitet und die Lösungen (a) und (b), an der Oberfläche der Membran vorbeiführt.

2. Verfahren nach Anspruch 1, dadurch gekennzeichnet, daß die als (a) bezeichnete Lösung das Salz einer Säure mit einem $pK_S$-Wert von > 2 oder das Salz einer Base mit einem $pK_B$-Wert von > 2 enthält.

3. Verfahren nach Anspruch 1, dadurch gekennzeichnet, daß man die wäßrige Lösung eines Salzes einer organischen Säure oder Base verwendet, das eine Löslichkeit in Wasser von mindestens 5 Gew.% aufweist.

**EP 0 200 106 B1**

4. Verfahren nach Anspruch 1,_dadurch gekennzeichnet,_ daß man als wäßrige Lösung eines Salzes einer organischen Säure die Lösung eines Alkalimetall- oder Erdalkalimetalsalzes einer Aminosäure oder Hydroxycarbonsäure verwendet.

5. Verfahren nach Anspruch 1, _dadurch gekennzeichnet,_ daß man als wäßrige Lösung eines Salzes einer Base die mineralsaure Lösung einer Aminosäure verwendet.

6. Verfahren nach Anspruch 1, _dadurch gekennzeichnet,_ daß man als wäßrige Lösung eines Salzes einer Base die Lösung eines sauren Salzes aus einer quaternären Ammoniumverbindung und einer mehrbasischen Mineralsäure verwendet.

**Claims**

1. A process for obtaining an aqueous solution of an organic acid or base, wherein

c) an aqueous solution of a salt of an organic acid or of an organic base and

b) an aqueous solution of an inorganic base or of an acid are each passed through compartements of an exchange cell which are separated from one another by an ion exchange membrane, and the solutions (a) and (b) are transported past the surface of the membrane.

2. A process as claimed in claim 1, wherein the solution referred to as (a) contains a salt of an acid having a $pK_A$ of > 2 or a salt of a base having a $pK_B$ of > 2.

3. A process as claimed in claim 1, wherein an aqueous solution of a salt of an organic acid or base is used, the said salt having a water-solubility of not less than 5% by weight.

4. A process as claimed in claim 1, wherein a solution of an alkali metal or alkaline earth metal salt of an amino acid or hydroxycarboxylic acid is used as the aqueous solution of a salt on an organic acid.

5. A process as claimed in claim 1, wherein a solution of an amino acid containing a mineral acid is used as the aqueous solution of a salt of a base.

6. A process as claimed in claim 1, wherein a solution of an acidic salt of a quaternary ammonium compound and a polybasic mineral acid is used as the aqueous solution of a salt of a base.

**Revendications**

1. Procédé de préparation de solutions aqueuses d'acides ou de bases organiques, caractérisé en ce qu'on fait passer respectivement

a) une solution aqueuse d'un sel d'un acide organique ou d'une base organique et

b) une solution aqueuse d'une base minérale ou d'un acide dans les chambres séparées l'une de l'autre par une membrane échangeuse d'ions d'une cellule d'échange et on amène les solutions (a) et (b) à la surface des membranes.

2. Procédé selon la revendication 1, caractérisé en ce que la solution désignée par (a) contient le sel d'un acide ayant un $pK_A$ > 2 ou le sel d'une base ayant un $pK_B$ > 2.

3. Procédé selon la revendication 1, caractérisé en ce qu'on utilise la solution aqueuse d'un sel d'un acide ou d'une base organique qui présente une solubilité dans l'eau d'au moins 5% en poids.

4. Procédé selon la revendication 1, caractérisé en ce qu'on utilise comme solution aqueuse d'un sel d'un acide organique la solution d'un sel de métal alcalin ou de métal alcalino-terreux d'un acide aminé ou d'un acide hydroxycarboxylique.

5. Procédé selon la revendication 1, caractérisé en ce qu'on utilise comme solution aqueuse d'un sel d'une base la solution dans un acide minéral d'un acide aminé.

6. Procédé selon la revendication 1, caractérisé en ce qu'on utilise comme solution aqueuse d'un sel d'une base la solution d'un sel acide d'un composé d'ammonium quaternaire et d'un polyacide minéral.